# EUROPÄISCHE PATENTSCHRIFT

(11) **EP 0 983 080 B1**
(45) Veröffentlichungstag und Bekanntmachung des Hinweises auf die Patenterteilung: **29.09.2004**
(21) Anmeldenummer: 97933617.9
(22) Anmeldetag: 26.05.1997
(51) Int. Cl.: A61K 38/17, A61K 7/48, A61K 7/04, A61K 7/06

(54) **SYNTHETISCHE, STATISTISCHE THYMUSPEPTID-KOMBINATION UND DEREN VERWENDUNG ALS IMMUNOLOGISCH UND/ODER ENDOKRINOLOGISCH WIRKENDES PRAPARAT**
SYNTHETIC, STATISTIC THYMUS PEPTIDE COMBINATION AND ITS USE AS A PREPARATION WITH IMMUNOLOGICAL AND/OR ENDOCRINOLOGICAL EFFECT
COMBINAISON STATISTIQUE SYNTHETIQUE DE PEPTIDES DE THYMUS ET SON UTILISATION COMME PREPARATION A EFFET IMMUNOLOGIQUE OU ENDOCRINIEN

(43) Veröffentlichungstag der Anmeldung: 08.03.2000
(73) Patentinhaber: Klett-Loch, Lore Maria, 68309 Mannheim (DE)
(72) Erfinder: Klett-Loch, Lore Maria, 68309 Mannheim (DE)
(74) Vertreter: Naumann, Ulrich, Dr.-Ing.
(86) Internationale Anmeldenummer: PCT/DE1997/001061
(87) Internationale Veröffentlichungsnummer: WO 1998/053845

(56) Entgegenhaltungen:
- EP-A- 0 237 398
- WO-A-96/17584
- CORDERO O J ET AL: "Novel approaches to immunotherapy using thymic peptides" IMMUNOLOGY TODAY, Bd. 18, Nr. 1, Januar 1997, Seite 10-13 XP004016755

## Beschreibung

Die Erfindung betrifft eine synthetische, statistische Thymuspeptid-Kombination und deren Verwendung als immunologisch und/oder endokrinologisch wirkendes Präparat.

Aus einer zunehmenden Zahl wissenschaftlicher Veröffentlichungen wird deutlich, daß es eine Kommunikation mittels niedermolekularer Signalstoffe zwischen dem Gehirn, den Organen des Immunsystems (Knochenmark, Thymus, Lympfknoten, Milz, Darmschleimhaut, Lungenepitel und Körperhaut) und dem endokrinen System der hormonalen Steuerung gibt. Aus Thymusgewebe wurden niedermolekulare Eiweißstoffe, sogenannte Signalpeptide identifiziert, die sowohl auf neuronaler Ebene als Neurotransmitter fungieren, als auch auf immunologischer Ebene als Differenzierungsfaktoren.

Es ließ sich in Tierexperimenten nachweisen, daß niedermolekulare Signalpeptide die Differenzierungskaskade in T- und B-Lymphozyten aus pluripotenten Stammzellen induzieren können, wenn derartige Peptidwirkstoffe subcutan dem Organismus zugeführt werden. Darüber hinaus ist ermittelt worden, daß derartige Signalmoleküle, deren Zusammensetzung proteinischen Elementen der Thymusdrüse entspricht, den Organismus vor opportunistischen Infektionen schützen können, wenn dieser z.B. durch Röntgenstrahlen, Krebs-Chemotherapie oder eine latente Virusinfektion vorgeschädigt ist. Begleitet werden derartige Vorschädigungen des regulatorischen Gleichgewichts des Organismus' durch hormonale Störungen, die sich hauptsächlich durch veränderte Fertilität, geistige und körperliche Leistungsminderung, Schlafstörungen und Appetitlosigkeit ausdrücken. Darüber hinaus fällt beim Menschen eine verminderte Schutzfunktion der Haut sowie der Qualität des Haar- und Nagelwuchses auf.

Erfahrungsheilkundlich wird diesem Syndrom seit langem dadurch begegnet, daß Organextraktpräparate als Arzneimittel oder Nahrungszusatzstoffe verwendet werden. Derartige Präparate aus tierischem Thymusgewebe, insbesondere Kalbsthymus, haben eine breite Verwendung gefunden, wenn auch bisher an solchen Präparaten ein molekular definiertes Wirkprinzip nicht beschrieben worden ist.

WO 96 17584 A bechreibt orale und topisch zu verabreichende Wirkstoffkombinationen zum Zwecke der Förderung des Wachstums von Haaren, Haut und Nägeln, wobei teilsynthetisch hergestellte Thymus-Ppetide enthalten sein können.

Oscar J. Cordero et al. beschreiben in "Immunology Today, bd. 18, no. 1, 1997" die

Verwendung von Thymuspeptiden zur Behandlung von congenitaler Immunschwäche.

Positive Daten aus einer Reihe von biologischen Studien *in vivo* mit solchen Thymuspräparaten erlauben den Schluß, daß Inhaltsstoffe des Thymusgewebe synergistische Effekte sowohl auf das neuronale wie auch das Immunsystem ausüben. Allerdings trat in den vergangenen fünf Jahren das Risiko der Kontamination solcher tierischen Organextrakt-Präparate mit Prion-Proteinen in den Vordergrund, denen angelastet wird, die Symptome des BSE (Bovine Spongiforme Encephalopathie, "Rinderwahnsinn") zu übertragen.

Demzufolge liegt der vorliegenden Erfindung die Aufgabe zugrunde, ein immunologisch und/oder endokrinologisch wirkendes Präparat bereitzustellen, das einerseits die positiven Erfahrungen mit Thymuspeptid-Kombinationen fortzuschreiben erlaubt, andererseits aber das BSE-Risiko gänzlich ausschließt. Des weiteren soll eine synthetische, statistische Thymuspetid-Kombination und deren Verwendung als immunologisch und/oder endokrinologisch wirkendes Präparat mit entsprechenden Vorteilen angegeben werden.

Die voranstehende Aufgabe wird erfindungsgemäß durch die Merkmale des Anspruches 1 gelöst. Danach enthält ein immunologisch und/oder endokrinologisch wirkendes Präparat.als Wirkstoff kurzkettige Peptide mit einem für Thymusgewebe charakteristisch gewichteten Mengenverhältnis und Muster an Aminosäuren.

Patentanspruch 2 bezieht sich auf ein entsprechendes Verfahren zur Herstellung, nämlich die schrittweise Aneinanderreihung von einzelnen, chemisch geeignet derivatisierten Aminosäuren zu kurzkettigen Peptiden und Freisetzung dieser synthetischen Peptide aus ihrer N-terminalen Gruppe und an Seitenfunktionen derivatisierten Form unter Aufrechterhaltung des für Thymusgewebe charakteristisch gewichteten Mengenverhältnisses und Musters an Aminosäuren.

Patentanspruch 3 betrifft die Verwendung entsprechender Präparate; d.h. von synthetisierten kurzkettigen Peptiden mit einem für Thymusgewebe charakteristisch gewichteten Mengenverhältnis und Muster an Aminosäuren mit immunologisch und/oder endokrinologisch wirksamen Eigenschaften, und zwar von synthetische, statistische Thymuspeptid-Kombinationen enthaltenden Präparaten. Die weiter abhängigen Patentansprüche 4 bis 8 beziehen sich auf ganz besondere Verwendungen, nämlich bei Immunschwäche und Immundefekten, bei hormonalen Störungen, als Baustoff für Haut, Haare und Nägel, im kosmetischen Bereich und schließlich im Bereich der Nahrungsergänzungsmittel.

Ausgehend von Thymusprotein-Partialhydrolysaten, Eiweißgemischen aus kurzkettigen Thymuspeptiden und -Aminosäuren, die durch enzymatische oder/und chemische Partialhydrolyse von Thymusgewebe entstehen, wurden derartige Kombinationen chromatographisch und aminosäureanalytisch dahingehend aufgeklärt, daß solche Partialhydrolysate aus kurzkettigen Peptiden bestehen, mit einem für Thymusgewebe charakteristischen Mengenverhältnis und Muster an Aminosäuren. Bringt man derartige Aminosäuren in chemisch geeignet derivatisierter Form miteinander zur Reaktion, wobei das für Thymusgewebe charakteristische Mengenverhältnis der Aminosäuren aufrecht erhalten wird, befreit derartige synthetische Kombinationen von ihren N-terminalen Schutzgruppen und kuppelt erneut und wiederholt mit derartig gewichteten Kombinationen aus Aminosäuren, die für Thymusgewebe charakteristisch sind, so entstehen synthetische, statistische Thymuspeptid-Kombinationen unter vollständigem Ausschluß des Prion-Protein-Risikos.

Die Erfindung offenbart somit relativ einfache Wege zur gezielten Herstellung von immunologisch und endokrinologisch wirksamen Präparaten mit erstmals standardisierbarer Präparatwirkung.

Derartige Thymuspeptid-Kombinationen lassen sich in pharmazeutisch reproduzierbarer Weise steril produzieren, so daß auf diese Weise ein Präparat entsteht, das je nach Anzahl der wiederholten, statistischen Syntheseoperationen aus einer Familie von Thymus-Signalpeptiden besteht, charakteristisch für Neurotransmitter und immunologisch aktive Signalmoleküle. Anhand analytischer Untersuchungen im Vergleich zu Partialhydrolysegemischen aus natürlichem Thymusgewebe konnte gezeigt werden, daß die synthetischen-statistischen Thymuspeptid-Kombinationen ihrer Zusammensetzung nach dem Naturpräparat innerhalb der Fehlergrenze der angewendeten analytischen Methoden vergleichbar sind. Daraus folgt, daß die erfindungsgemäße Aufgabe, das BSE-Risiko durch Synthese einer Thymuspeptid-Kombination zu beseitigen, erfüllt werden konnte.

Es hat sich überraschenderweise gezeigt, daß das erfindungsgemäße synthetische Präparat im biologischen Test an Human-Lymphozyten überlegene Aktivität bezüglich der mitogenen Kostimulation (Induktion der Zellvermehrung) im Vergleich zu einem marktüblichen Thymus-Partialhydrolysepräparat aufweist.

Es ist weiterhin überraschend, daß das synthetische Präparat Bestandteile der extrazellulären Matrix zu binden vermag, Eigenschaft, die bisher nur Skleroproteinen zuzuschreiben war.

Die Erfindung wird durch nachfolgende Beispiele näher erläutert.

### Beispiel 1:

Die erfindungsgemäße statistische Thymuspeptid-Kombinationen wurde im Testsystem der Mitogenen Kostimulation im Vergleich zu einem marktüblichen Thymus-Partialhydrolysepräparat auf Bioaktivität hin untersucht. Getestet wurden Endkonzentrationen von 100, 50 und 25 µg/ml.

Bei zwei Spendem (KFG und WH) zeigte die statistische Thymuspeptid-Kombination eine statisch signifikant höhere Bioaktivität als ein marktübliches Thymus-Partialhydrolysepräparat. Während für ein marktübliches Thymus-Partialhydrolysepräparat bei der hohen Konzentration von 100 µg/ml eine Inhibition der Zellproliferation beobachtet wurde, führte die statistische Thymuspeptid-Kombination zu einer dosisabhängigen Steigerung der Proliferation von humanen Lymphozyten.

Humane periphere Blutlymphozyten wurden aus zwei Blutspenden isoliert, und die Proliferationsrate dieser Zellen nach suboptimaler Vorstimulation (0,5 µg/ml Phytohämagglutinin, PHA) unter der Wirkung verschiedener Konzentrationen von einem marktüblichen Thymus-Partialhydrolysepräparat und der statistischen Thymuspeptid-Kombination im Vergleich zu der Positivkontrolle REF01 (Thymosin Fraktion 5) gemessen.

### Meßtechnisch wurde die Proliferationsrate nach folgendem Verfahren ermittelt:

Bei der Wirkstoffabhängigen Induktion der T-Zellproliferation wird die DNA-Synthese zum Zweck der Zellteilung angeregt, daher kann über den Einbau eines radioaktiven DNA-Bausteins (³H-Thymidin) die T-Zellvermehrung anhand eingebauter Radioaktivität gemessen werden.

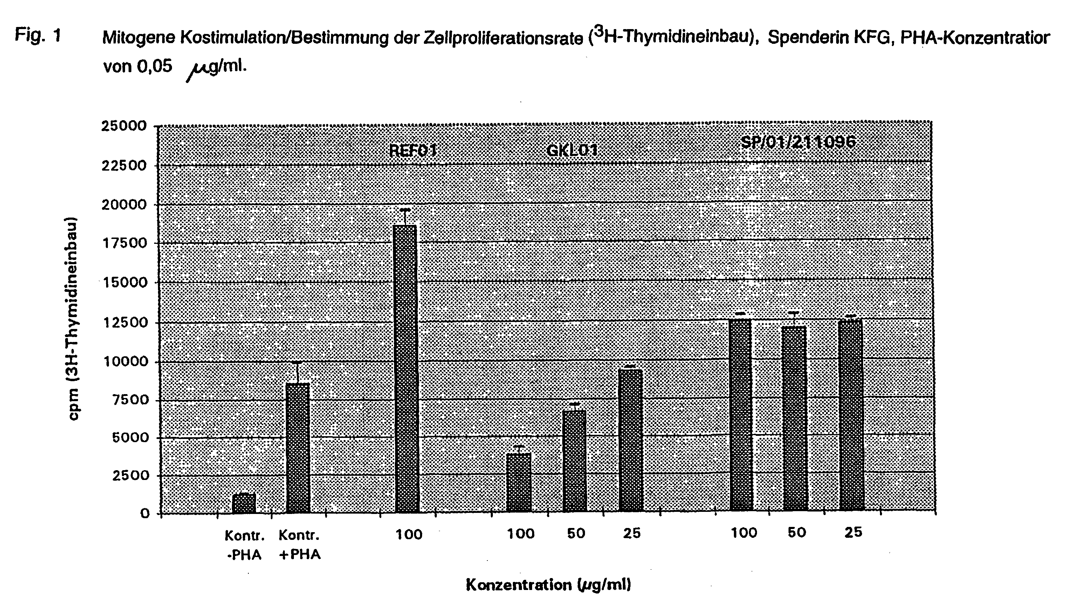

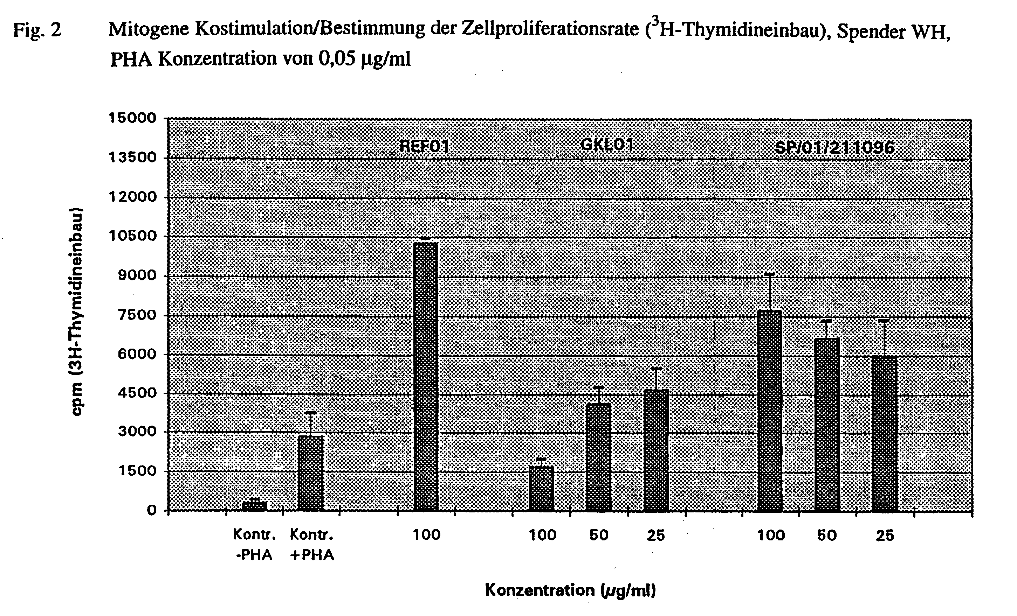

Die Figuren 1 und 2 zeigen Ergebnisse aus der Stimulation der Blutlymphozyten der Spenderin KFG und des Spenders WH aufgeführt. Die Versuchsdaten sind auf das Resultat des Kontrollansatzes nach Zusatz von Phytohämagglutinin (Kontr. + Phytohämagglutinin) zu beziehen. Die Positivkontrolle Referenzthymuspräparat wurde in einer Konzentration von 100µg/ml im Test eingesetzt; ein marktübliches Thymus-Partialhydrolysepräparat und die statistische Thymuspeptid-Kombination wurden in den drei Konzentrationen 100, 50 und 25µg/ml getestet.

Fig. 1 zeigt die Ergebnisse für die Spenderin KFG. Die Positivkontrolle Referenzthymuspräparat führte zu einer sehr deutlichen Steigerung der Proliferationsrate. Für ein marktübliches Thymus-Partialhydrolysepräparat dagegen konnte eine dosisabhängige Inhibition beobachtet werden; erst bei der niedrigsten Konzentration von 25 µg/ml wurde der Wert des Kontrollansatzes erreicht. Unter der Wirkung der statistischen Thymuspeptid-Kombination lag die Zellproliferationsrate bei den drei getesteten Konzentrationen statistisch signifikant höher als im Kontrollansatz.

Vergleichbare Ergebnisse zeigten sich mit Lymphozyten des Spenders WH gemäß Fig. 2. Wiederum ergab die Positivkontrolle Referenzthymuspräparat eine starke Zunahme der Proliferation von humanen Lymphozyten. Ein marktübliches Thymus-Partialhydrolysepräparat führte in den Konzentrationen von 50 und 25µg/ml zu einer geringen Erhöhung der Zellproliferationrate, nicht dagegen in der hohen Konzentration von 100 µg/ml. Dagegen konnte für die statistische Thymuspeptid-Kombination SP/01/211096 eine deutliche und dosisabhängige Steigerung der DNA-Syntheserate beobachtet werden.

Ziel dieser Untersuchungen gemäß Beispiel 1 war die vergleichende Testung der syntetisch-statistischen Thymuspeptid-Kombination mit einem marktüblichen Thymus-Partialhydrolysepräparat im Testsystem der Mitogenen Kostimulation. Untersucht werden sollte die Fähigkeit der Prüfsubstanz zur Steigerung der Zellproliferationsrate von humanen Blutlymphozyten.

Für die statistische Thymuspeptid-Kombination konnte bei zwei Spendem eine deutlich höhere Bioaktivität im Vergleich zu marktüblichem Thymus-Partialhydrolysepräparat festgestellt werden. Beim Spender WH ergab sich zudem eine dosisabhängige Erhöhung der DNA-Syntheserate. Überraschenderweise führte der Zusatz von einem marktüblichen Thymus-Partialhydrolysepräparat zu den Zellkulturansätzen eher zu einer Inhibition der Zellproliferation. Nur bei niedrigen Konzentrationen von 50 bzw. 25µg/ml lag die Zellproliferationsrate beim Spender WH geringfügig über dem Wert des Kontrollansatzes.

### Beispiel 2:

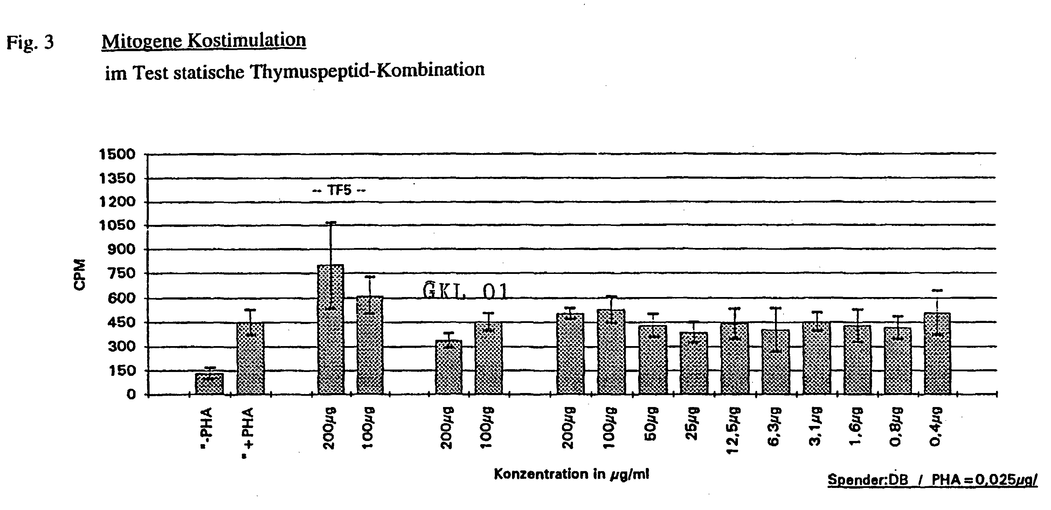

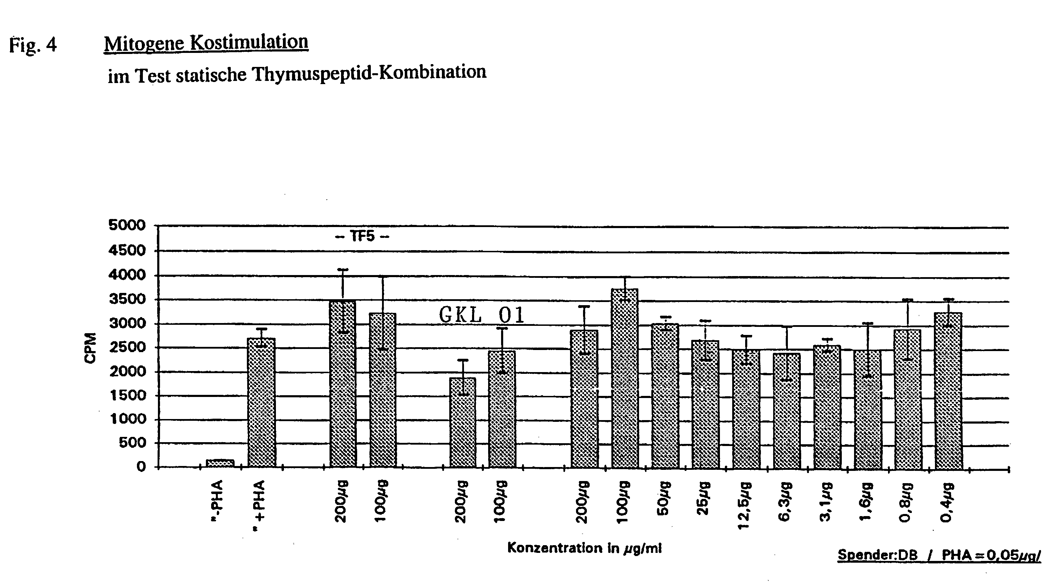

Das nachfolgende Beispiel bezieht sich auf die Stimulation der Blutlymphozyten des Spenders DB. Die Versuchsdaten sind unter Bezugnahme auf die Figuren 3 und 4 auf das Resultat des Kontrollansatzes nach Zusatz von Phytohämagglutin in PHA zu beziehen. Als Positivkontrolle diente Thymosin-Fraktion Nr. 5 (TF5) in zwei Konzentrationen (3. und 4. Kolonne von links). Zum Vergleich wurde auch in gleicher Konzentration ein marktübliches Thymus-Partialhydrolysat eingesetzt (5. und 6. Kolonne). Als Kontrolle wurde PHA alleine eingesetzt.

Die synthetisch-statistische Thymuspeptid-Kombination weist in der mitrogenen Kostimulation bei suboptimaler Stimulation der Blutzellen mit PHA von 0,05 µg sowohl bei einer Dosierung des beanspruchten Syntheseproduktes von 100 µg/Zellkulturansatz als auch bei 0,4 µg/Zellkulturansatz eine überraschend ausgeprägte Aktivität auf.

Bei geringer Vorstimulation der Blutzellen mit PHA, 0,025 µg/Ansatz sind die Wirkungen der erfindungsgemäßen synthetisch-statistischen Thymuspeptid-Kombination ebenfalls deutlich zu erkennen, wenn auch nicht so ausgeprägt wie bei einer höheren Vorstimulation der Blutzellen mit 0,05µg PHA/Ansatz.

Letztendlich liegt hier eine typische in vitro, zweiphasige Aktivität vor, die im Bereich von 100µg/ml und 0,4µg/ml jeweils ein Maximum zeigt, und jeweils besser als das Vergleichspräparat dienende marktübliche Thymus-Partialhydrolysat. Diese zweiphasige Aktivität ist jedenfalls ein Beleg für den breiten Umfang der immunologischen in vitro Aktivität, wobei das hohe Maß an Aktivität bei der extrem niedrigen Konzentration von 0,4 µg/ml zweifelsohne überrascht und aufgrund der wirklich sehr niedrigen Konzentration von großem wirtschaftlichem Interesse ist.

Die analytischen Daten der synthetisch-statistischen Thymuspeptid-Kombination ergeben sich wie folgt:

| **Certificate of Analysis** **Data sheet Synth. statistic Thymic Peptides** | | |
|---|---|---|
| **Description:** | **Specifications** **Specified** yellowish powder | # SP/01/211096 **Found** yellowish powder |
| **Residual solvents (GC):** | | |
| Water | ≤ 10 % | 9 % ± 0.5 % |
| DMF | ≤ 0,1 % | n.d. * <200ppm** |
| Methanol | ≤ 0,1 % | 130 ppm <130ppm |
| Toluol | ≤ 0,1 % | n.d. <40ppm |
| Ethylisopropylamin | ≤ 0,1 % | n.d. <200ppm |
| Essigsäureethylester | ≤ 0,1 % | n.d. <20ppm |
| | | |
| **Acetate:** | ≤ 3 % | 1.6 % ± 0,2 % |
| | | |
| Inorganic salt: | ≤ 0.5 % | < 0.2 % |
| | | |
| **Specific rotation:** (20°C c=0.2; H₂O) | -30.0° ± 5° | -30.2° ± 0.2° |
| | | |
| **Free Amino Acid Content:** (%, w/w) | GKL01 ± 5 % | complies |
| | | |
| **Total Amino Acid Composition:** (%, w/w) | GKL01 ± 5 % | complies |
| | | |
| **Peptide Content:** | 50 % ± 5 % | complies |

## Patentansprüche

1. Vollsynthetische Thymuspeptid-Kombinationen enthaltendes immunologisch und/oder endokrinologisch wirkendes Präparat ohne BSE-Risiko erhältlich durch schrittweise Aneinanderreihung von einzelnen, chemisch geeignet derivatisierten Aminosäuren zu kurzkettigen Peptiden und Freisetzung dieser synthetischen Peptide aus ihrer N-terminalen Gruppe und an Seitenfunktionen derivatisierten Form unter Ausbildung des für Thymusgewebe charakteristisch gewichteten Mengenverhältnisses und Musters an Aminosäuren, wobei das Präparat eine dosisabhängige Steigerung der Proliferation von humanen Lymphozyten in Endkonzentrationen von 100, 50 und 25 µg/ml aufweist.

2. Synthetische-statistische Thymuspeptid-Kombinationen enthaltendes Präparat nach Anspruch 1,
**dadurch gekennzeichnet, dass** keine Inhibition der Zellproliferation bei einer Konzentration von 100 µg/ml vorhanden ist.

3. Synthetische-statistische Thymuspeptid-Kombinationen enthaltendes Präparat nach Anspruch 1 oder 2 als endokrinologisch und/oder immunologisch wirksames Mittel.

4. Synthetische-statistische Thymuspeptid-Kombinationen enthaltendes Präparat nach Anspruch 1 oder 2 zur Verwendung als Arzneimittel bei der Behandlung von Immunschwächen und Immundefekten.

5. Synthetische-statistische Thymuspeptid-Kombinationen enthaltendes Präparat nach Anspruch 1 oder 2 zur Verwendung als Arzneimittel bei der Behandlung von hormonalen Störungen.

6. Synthetische-statistische Thymuspeptid-Kombinationen enthaltendes Präparat nach Anspruch 1 oder 2 als Aufbaustoff für Haut, Haare, Nägel.

7. Synthetische-statistische Thymuspeptid-Kombinationen enthaltendes Präparat nach Anspruch 1 oder 2 als kosmetisches Mittel.

8. Synthetische-statistische Thymuspeptid-Kombinationen enthaltendes Präparat nach Anspruch 1 oder 2 als Nahrungsergänzungsmittel.

9. Verfahren zur Herstellung eines Präparats nach einem der Ansprüche 1 bis 8, wobei einzelne, chemisch geeignet derivatisierte Aminosäuren zu kurzkettigen Peptiden aneinandergereiht werden und Freisetzung dieser synthetischen Peptide aus ihrer N-terminalen Gruppe und an Seitenfunktion derivatisierten Form,
**dadurch gekennzeichnet, dass** das für Thymusgewebe charakteristisch gewichtete Mengenverhältnis und Muster an Aminosäuren ausgebildet wird.

## Claims

1. Immunologically and/or endocrinologically active preparation containing fully synthetic thymic peptide combinations, without a risk of BSE, which preparation is obtainable by lining up in steps individual chemically suitably derivatised amino acids to form short-chain peptides and releasing those synthetic peptides from their N-terminal group and form derivatised on side functions, with the formation of the quantitative ratio and pattern of amino acids weighted characteristically for thymus tissue, the preparation having a dose-dependent increase in the proliferation of human lymphocytes in final concentrations of 100, 50 and 25 µg/ml.

2. Preparation containing synthetic-statistical thymic peptide combinations according to claim 1, **characterised in that** there is no inhibition of cell proliferation at a concentration of 100 µg/ml.

3. Preparation containing synthetic-statistical thymic peptide combinations according to claim 1 or 2, as an endocrinologically and/or immunologically active agent.

4. Preparation containing synthetic-statistical thymic peptide combinations, according to claim 1 or 2, for use as a medicament in the treatment of immune weaknesses and immunodeficiencies.

5. Preparation containing synthetic-statistical thymic peptide combinations, according to claim 1 or 2, for use as a medicament in the treatment of hormonal disorders.

6. Preparation containing synthetic-statistical thymic peptide combinations, according to claim 1 or 2, as a building substance for skin, hair, nails.

7. Preparation containing synthetic-statistical thymic peptide combinations, according to claim 1 or 2, as a cosmetic agent.

8. Preparation containing synthetic-statistical thymic peptide combinations, according to claim 1 or 2, as a food supplement.

9. Process for the manufacture of a preparation according to any one of claims 1 to 8, individual, chemically suitably derivatised amino acids being lined up to form short-chain peptides, and releasing those synthetic peptides from their N-terminal group and form derivatised on side functions, **characterised in that** the quantitative ratio and pattern of amino acids weighted characteristically for thymus tissue are formed.

## Revendications

**1.** Préparation agissant immunologiquement et/ou endocrinologiquement contenant des combinaisons totalement synthétiques de peptides de thymus sans risque d' ESB, pouvant être obtenue par mise à la suite les uns avec les autres pas à pas d'acides aminés individuels dérivés chimiquement de manière appropriée en peptides à chaîne courte et libération de ces peptides synthétiques de leur groupe N-terminal et dans une forme dérivée en fonctions secondaires en formant la constitution quantitative et l'échantillon pondérés en acides aminés caractéristiquement pour le tissu de thymus, la préparation présentant une croissance dépendant de la dose de la prolifération de lymphocytes humains dans des concentrations finales de 100, 50 et 25 µg/ml.

**2.** Préparation contenant des combinaisons synthétiques statistiques de peptides de thymus selon la revendication 1,
**caractérisée par le fait qu'**il n'y a pas présence d'inhibition de la prolifération cellulaire pour une concentration de 100 µg/ml.

**3.** Préparation contenant des combinaisons synthétiques statistiques de peptides de thymus selon la revendication 1 ou 2 en tant qu'agent actif endocrinologiquement et/ou immunologiquement.

**4.** Préparation contenant des combinaisons synthétiques statistiques de peptides de thymus selon la revendication 1 ou 2 pour l'utilisation en tant que médicament pour le traitement d'asthénies immunitaires et de défaillances immunitaires.

**5.** Préparation contenant des combinaisons synthétiques statistiques de peptides de thymus selon la revendication 1 ou 2 pour l'utilisation en tant que médicament pour le traitement de troubles hormonaux.

**6.** Préparation contenant des combinaisons synthétiques statistiques de peptides de thymus selon la revendication 1 ou 2 en tant que matériau de constitution pour de la peau, des cheveux, des ongles.

**7.** Préparation contenant des combinaisons synthétiques statistiques de peptides de thymus selon la revendication 1 ou 2 en tant qu'agent cosmétique.

**9.** Procédé pour la fabrication d'une préparation selon l'une des revendications 1 à 8, des acides aminés individuels dérivés chimiquement de manière appropriée en peptides à chaîne courte étant mis à la suite les uns avec les autres et libération de ces peptides synthétiques de leur groupe N-terminal et dans une forme dérivée en fonctions secondaires,
**caractérisé par le fait que** la constitution quantitative et l'échantillon pondérés en acides aminés caractéristiquement pour le tissu de thymus sont formés pour le tissu de thymus.
